(19) Europäisches Patentamt / European Patent Office / Office européen des brevets

(11) **EP 2 861 216 B1**

(12) **FASCICULE DE BREVET EUROPEEN**

(45) Date de publication et mention de la délivrance du brevet:
**17.04.2019 Bulletin 2019/16**

(21) Numéro de dépôt: **13729029.2**

(22) Date de dépôt: **14.06.2013**

(51) Int Cl.:
*A61K 9/107* *(2006.01)*   *A61K 31/197* *(2006.01)*
*A61K 9/50* *(2006.01)*   *A61K 47/10* *(2017.01)*
*A61K 47/12* *(2006.01)*   *A61K 47/14* *(2017.01)*
*A61K 9/00* *(2006.01)*   *A61K 9/16* *(2006.01)*
*A61K 9/20* *(2006.01)*   *A61K 9/48* *(2006.01)*

(86) Numéro de dépôt international:
**PCT/EP2013/062398**

(87) Numéro de publication internationale:
**WO 2013/186370 (19.12.2013 Gazette 2013/51)**

(54) **FORMULATION PHARMACEUTIQUE ORALE DE MOLECULES BCS DE CLASSE III**

ORALE PHARMAZEUTISCHE FORMULIERUNG MIT BCS-KLASSE III-MOLEKÜLEN

ORAL PHARMACEUTICAL FORMULATION OF BCS CLASS III MOLECULES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.06.2012 FR 1255593**

(43) Date de publication de la demande:
**22.04.2015 Bulletin 2015/17**

(73) Titulaire: **Ethypharm**
**92210 Saint-Cloud (FR)**

(72) Inventeurs:
• **AILHAS, Caroline**
  **27460 Alizay (FR)**
• **HERRY, Catherine**
  **27670 Saint-Ouen du Tilleul (FR)**

(74) Mandataire: **Regimbeau**
**20, rue de Chazelles**
**75847 Paris Cedex 17 (FR)**

(56) Documents cités:
EP-A1- 1 254 659    WO-A1-2005/025559
US-A1- 2010 029 771

EP 2 861 216 B1

**Description**

L'invention concerne une formulation galénique orale de molécule BCS de classe III, le baclofène, présentant une biodisponibilité et une perméabilité améliorées.

La voie orale est la voie envisagée en premier lieu lors de la mise en forme d'une nouvelle entité pharmaceutique de par l'observance au traitement qu'elle induit. Cependant, cette voie est abandonnée pour de nombreuses molécules en développement en raison de leur faible biodisponibilité orale. Celle-ci peut être due à divers facteurs provenant des propriétés même de la molécule ou bien de la physiologie du tractus gastro-intestinal *(Fasinu, et al., 2011)*. Diverses approches ont été explorées ces dix dernières années pour améliorer la biodisponibilité orale de ces molécules incluant des moyens physiques ou chimiques.

La classification de molécules BCS (Biopharmaceutics Classification System) est un outil significatif utilisé pour le développement des formes orales dans l'industrie pharmaceutique et est notamment adopté par la FDA, l'EMEA et l'OMS *(Dahan, et al., 2009)*. Celle-ci se divise en quatre catégories de molécules et se base sur les éléments fondamentaux qui contrôlent l'absorption orale que sont la perméabilité membranaire intestinale ainsi que la solubilité d'une molécule dans le milieu gastro-intestinal. Une molécule est considérée soluble si la dose maximale d'une forme à libération immédiate est soluble dans 250 ml ou moins d'un milieu aqueux dont le pH est compris entre 1,2 et 6,8 et est considérée perméable si son absorption à travers la membrane intestinale est supérieure ou égale à 90 %. Une molécule BCS appartenant à la classe III est hautement soluble et faiblement perméable. Cette dernière particularité est le facteur limitant la biodisponibilité orale et peut mener à l'abandon du développement de formulation orale de molécules ayant pourtant un fort potentiel thérapeutique.

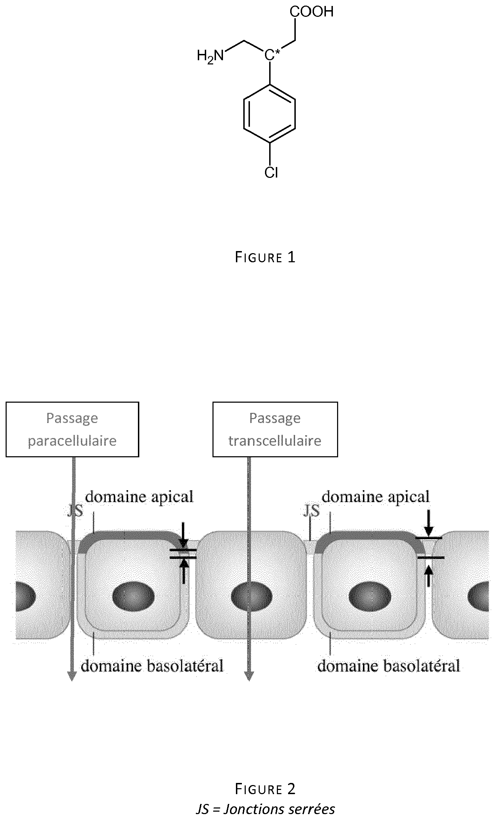

Le baclofène (figure 1) est une molécule BCS appartenant à la classe III. Ses caractéristiques physico-chimiques sont résumées dans le tableau 1.

*TABLEAU 1 : CARACTERISTIQUES PHYSICO-CHIMIQUES DU BACLOFENE (RAPPORT INTERNE)*

| Masse molaire | 213.66 g/mol |
|---|---|
| Point de fusion | 180-191°C |
| pH d'une solution saturée | 6,5 |
| pKa | $pKa_1 = 3.87$<br>$pKa_2 = 9.62$ |
| Solubilités | • <u>Soluble</u> : HCl 0.1N, NaOH 0.1N<br>• <u>Légèrement soluble</u> : eau (3 mg/ml)<br>• <u>Très légèrement soluble</u> : Ethanol, Méthanol |
| Log P | -1 |

Aux doses usuelles thérapeutiques, le baclofène est connu pour avoir une bonne biodisponibilité orale. Pourtant son faible log P indique une certaine hydrophilie et donc une faible perméabilité. Cette observation s'explique par la présence de transporteurs spécifiques au niveau de l'intestin grêle permettant le passage de la molécule. Celui-ci est d'autant plus important au niveau du jéjunum. Aussi, un faible passage au niveau du colon suppose la présence d'un autre mécanisme de passage à travers la barrière intestinale *(Merino, et al.,* 1989). Cette zone du système digestif ne contient pas de transporteurs mais la molécule étant hydrophile et de petite taille, un faible passage passif à travers les jonctions serrées est possible. Ainsi, il apparaît que lors d'un besoin en concentration plasmatique plus importante du baclofène, la prise d'une dose plus élevée n'est pas efficace en raison de la saturation des transporteurs. La solution utilisée en clinique est alors la prise de faibles doses à intervalles de temps rapprochés ce qui peut rapidement devenir contraignant pour le patient.

Une augmentation de la lipophilie du baclofène et par là-même de sa perméabilité transcellulaire a été observée grâce à la réalisation de prodrogues d'ester de baclofène *(Leisen, et al., 2003)*. De par ces propriétés, une plus grande concentration de prodrogue est retrouvée au niveau du tissu cible, le cerveau, grâce à un passage plus aisé de la barrière hémato-encéphalique. Cependant, il est à noter une affinité plus importante pour la pompe d'efflux P-gp ainsi qu'une hydrolyse partielle de la prodrogue en baclofène ce qui conduit finalement à un taux au niveau du site d'action plus bas en baclofène après une administration de la prodrogue par comparaison avec une administration de baclofène seul.

Une fenêtre d'absorption du baclofène est mise en évidence au niveau de l'intestin grêle en raison de la présence de transporteurs *(Merino, et al., 1989)*. Afin d'augmenter la biodisponibilité, cette fenêtre d'absorption pourrait ainsi être mise à profit par des techniques galéniques permettant la rétention de la forme orale au niveau de la fenêtre ou en amont *(Davis, 2005)*. En effet, un temps de résidence plus long au niveau du site d'absorption doit permettre en théorie un passage plus important de molécules à travers la barrière intestinale si celles-ci ne sont pas susceptibles de subir de dégradation présystémique avant absorption. Ainsi, des formes bioadhésives au mucus intestinal grâce à l'utilisation de polymères cationiques tels que le chitosane ou bien des formes gastrorétentives permettant le gonflement et la flottaison de la forme au niveau de l'estomac ont fait leur apparition ces dernières années en développement pharmaceutique mais leur efficacité est très dépendante de la variabilité intra et inter individuelle.

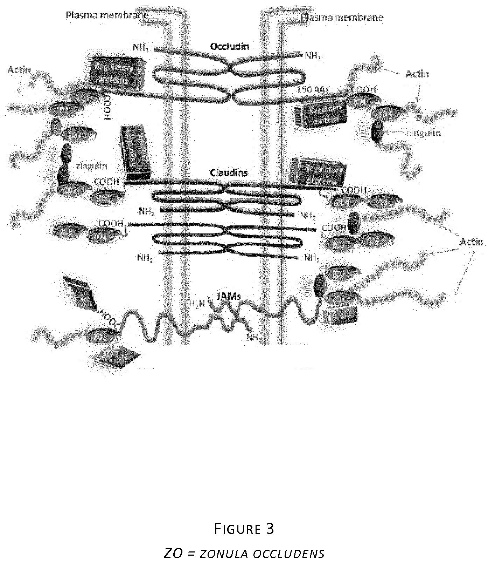

Dans des conditions physiologiques normales, le passage paracellulaire occupe moins de 0.1 % de la surface totale de l'épithélium intestinal donc n'est pas une voie de passage majoritaire *(Anilkumar, et al., 2001)*. Ceci s'explique par la présence des jonctions serrées entre les cellules qui limite l'absorption de molécules supérieures à 0,1 nm. Ainsi une action sur ces dernières pourrait permettre une augmentation d'absorption significative. Les jonctions serrées ont un rôle de barrière entre les composants des domaines apicaux et basolatéraux et sont composées de divers complexes de protéines impliqués dans la régulation de l'intégrité des jonctions (zonulaoccludens, actine, claudin-1, ...) (figures 2 et 3). Cette intégrité est modifiée face à divers agents physiologiques et pathologiques et notamment par des messagers secondaires provenant des voies de signalisation. Deux facteurs semblent mis en jeu face à l'action des promoteurs d'absorption : la contraction d'un anneau d'actine-myosine ainsi que la phosphorylation par des protéines kinases et phosphatases. *Anilkumar et al.* ont fait la liste des promoteurs d'absorption faisant l'objet de recherche dans cette indication. On trouve parmi eux des surfactants, des acides biliaires et dérivés, des acides gras et dérivés, des agents chélatants, le chitosane et ses dérivés, ainsi que le conjugué polycarbophyl-cystéine.

Ainsi, les jonctions serrées ayant un rôle de barrière et donc de défense de l'organisme, il est important que les promoteurs agissant sur leur ouverture aient une action réversible et n'induisent pas de toxicité.

La demande de brevet américain US2010/029771 décrit une formulation pharmaceutique liquide pouvant être intégrée dans une gélule pour le traitement de la gastroparésie et la dyspepsie non ulcéreuse avec des agonistes de l'acide gamma-aminobutyrique-B (GABAB) récepteurs, comme le baclofène par exemple.

La demande internationale WO2005/025559 décrit une formulation pharmaceutique liquide de type émulsion comprenant un principe actif tel que le baclofène dans la phase aqueuse et des agents émulsifiants dans la phase huileuse.De façon surprenante, les inventeurs ont découvert que la biodisponibilité, et plus particulièrement la perméabilité membranaire intestinale, des molécules BCS de classe III peut être considérablement améliorée par leur formulation sous forme d'une microémulsion huile-dans-eau, la molécule BCS de classe III étant aqueuse et la phase huileuse étant constituée d'un excipient huileux auto-émulsifiable au contact de l'eau.

En particulier, ils ont observé une augmentation de la perméabilité sur cellules Caco-2 du baclofène dans une formulation microémulsion huile-dans-eau entre une solution aqueuse de baclofène à 6 mg/mL dans 0,01 M d'acide fumarique et le Labrasol®.

Par conséquent, l'objet de l'invention concerne une formulation pharmaceutique orale à base d'une microémulsion entre une phase aqueuse comprenant au moins un principe actif BCS (Biopharmaceutics Classification System) de classe III, ledit principe actif étant le baclofène, et une phase huileuse comprenant un excipient huileux auto-émulsifiable au contact de l'eau.

La formulation selon l'invention présente les avantages d'une perméabilité membranaire intestinale améliorée et de non toxicité.

On entend par microémulsion une émulsion dont la taille des gouttelettes est inférieure à 200 $\mu$m.

On entend par « un excipient huileux auto-émulsifiable au contact de l'eau » un excipient qui va spontanément former une émulsion huile-dans-eau avec une phase aqueuse, soit des gouttelettes de la phase aqueuse entourées d'une couche lipidique comprenant l'excipient.

Afin d'évaluer le potentiel d'augmentation de perméabilité d'un promoteur d'absorption, des techniques *in vivo* chez le rat ou *ex-vivo* (sur intestin de rat perfusé) peuvent être réalisées *(Koga, et al., 2002)(Lin, et al., 2007) (Constantinides, et al., 1996)*. Des techniques *in vitro* sont également employées sur membrane intestinale de rat ou sur cultures cellulaires. Dans une première approche, l'évaluation sur lignée cellulaire Caco-2 est très souvent employée *(Sha, et al., 2005)*.

Les cellules Caco-2 sont issues de culture de cellules humaine d'origine cancéreuse capable de différenciation en présence d'un milieu de culture adapté *(Pontier, 1997-1998)*. Leurs propriétés sont fonction du nombre de repiquages auxquels elles sont soumises. Le clone de cellules obtenu au passage 198 présente des caractéristiques proches des entérocytes. Les cellules Caco-2 ont une taille légèrement inférieure à celle des entérocytes sains mais présentent une monocouche comparable à celle de l'épithélium de l'intestin grêle. La bordure en brosse est différenciée et développée et des jonctions très étroites de 10 à 50 Å sont présentes (plus étroites que celles de l'intestin grêle). D'un point de vue biochimique, les cellules Caco-2 expriment également les enzymes des entérocytes. Par ailleurs, de nombreux transporteurs sont présents mais leur expression est inférieure aux taux retrouvés *in vivo* et la pompe d'efflux P-gp est surexprimée ce qui peut sous-estimer une absorption par rapport à l'*in vivo*.

Le test du MTT est un indicateur de l'intégrité et de l'activité mitochondriales et est assimilable à une mesure de la viabilité cellulaire *(Sigentec)*. Ce test est basé sur l'activité d'une enzyme mitochondriale, la succinate déshydrogénase. En présence du substrat MTT (3-[4,5-diméthylthiazol-2yl]-2,5-diphényltétrazolium bromide), les sels de tétrazolium du substrat sont transformés en cristaux insolubles de formazan grâce à l'activité de la succinate déshydrogénase. Après solubilisation, la quantité de sel formazan est ensuite dosée par spectrophotométrie et est comparée à un contrôle négatif ayant une viabilité cellulaire de 100 % *(Buyukozturk, et al., 2010)*.

La mesure de la résistance électrique transépithéliale (ou TEER) (en ohms/cm$^2$) permet d'évaluer l'intégrité cellulaire *(Pontier, 1997-1998)*. Ceci permet d'évaluer la toxicité du produit testé. La mesure se fait avant et après l'essai et la perte de résistance ne doit pas excéder 30 % pour conclure à une non toxicité *(Oroxcell, 2011)*.

La lucifer yellow (LY) est un marqueur de passage paracellulaire *(Buyukozturk, et al., 2010)*. Son dosage dans le compartiment receveur pendant l'essai de perméabilité permet d'observer une ouverture des jonctions serrées. Le test peut également se faire après l'essai pour évaluer l'intégrité cellulaire *(Nollevaux, et al., 2006)*. La perméabilité apparente de la luciferyellow dans des conditions normales est de 3.10$^7$ cm/s *(Oroxcell, 2011)*.

De préférence, le ratio principe actif BCS de classe III/excipient huileux auto-émulsifiable au contact de l'eau en poids est compris entre 1/10 à 1/100, de manière particulièrement préférée entre 1/40 et 1/80.

De préférence, la phase huileuse représente de 1 à 50 % de la micro-émulsion, de manière encore préférée de 2 à 30 %, de manière particulièrement préférée de 5 à 25 %, ou encore de 15 à 20 %.

De préférence, la phase aqueuse de la micro-émulsion de la formulation de l'invention est une solution dans laquelle le principe actif BCS de classe III est solubilisé.

De préférence, la micro-émulsion de la formulation de l'invention est une micro-émulsion huile-dans-eau constituée d'une phase aqueuse comprenant au moins un principe actif BCS (Biopharmaceutics Classification System) de classe III qui est le baclofène et d'un excipient huileux auto-émulsifiable au contact de l'eau.

Le principe actif de la formulation selon l'invention est le baclofène.

Selon un mode de réalisation préféré, il est solubilisé dans la phase aqueuse, de préférence dans de l'acide fumarique 0,01 M.

De préférence, un excipient huileux auto-émulsifiable au contact de l'eau approprié présente un indice HLB (Hydrophilic-Lypophilic Balance) supérieur à 12, de manière particulièrement préférée de 13 ou 14, par exemple il s'agit du Labrasol® *(Gattefossé)* composé de caprylocaproylmacrogolglycérides ou de la Gélucire® 50/13 semi-solide composée de Lauroylmacrogolglycérides (Gattefossé).

De manière particulièrement préférée, un excipient huileux auto-émulsifiable au contact de l'eau approprié est choisi dans le groupe constitué :

- des acides gras en C8 à C10, pégylés ou non, comme le caprate de sodium ou l'un de ses dérivés,
- des triglycérides en C8-C10, comme les triglycérides d'acide caprique (C10) et caprylique (C8), par exemple les *Miglyol et Captex® (Sasol Germany GmbH, 2012)*,
- des mélanges de mono-, bi- et triglycérides, par exemple les *Capmul® (Abitec, 2012)*,
- des mélanges de mono-, bi- et triglycérides d'acides gras estérifiés de propylène glycol, par exemple des acides caprique et caprylique, comme des caprylocaproylmacrogolglycérides ou un mélange de mono-, bi- et triglycérides d'acides gras estérifiés PEG-8 à raison de 50 à 80 % d'acide caprylique et 20 à 50 % d'acide caprique, par exemple le *Labrasol®*.

Les Miglyol® *(Sasol)* ainsi que les Captex® *(Abitec)* sont des triglycérides d'acide caprique (C10) et caprylique (C8). Le Capmul®MCM *(Abitec)* est composé de mono- et di-glycérides d'acide caprique et caprylique (Tableau 2).

Le Labrasol® *(Gattefossé)* est composé de caprylocaproylmacrogolglycérides, ce qui consiste en un mélange de mono-, bi- et triglycérides ainsi que d'acides gras estérifiés de propylène glycol (Gattefossé). Ses caractéristiques (Tableau 2), et notamment sa miscibilité dans l'eau, s'avèrent particulièrement intéressantes d'un point de vue de la formulation.

La Gélucire® 50/13 (Tableau 2) est un excipient semi-solide, possédant un HLB élevé de 13 composés de Lauroylmacrogolglycérides (Gattefossé). L'acide palmitique et l'acide stéarique sont les acides gras qui composent la Gélucire®.

De manière particulièrement préférée, l'excipient huileux auto-émulsifiable de la micro-émulsion de la formulation de l'invention est le Labrasol®, soit un mélange de caprylocaproylmacrogolglycérides qui consiste en un mélange de mono-, bi- et triglycérides d'acides gras estérifiés de propylène glycol -8, avec une proportion de 50 à 80 % d'acide caprylique et 20 à 50 % d'acide caprique. Un autre objet de l'invention concerne une formulation selon l'invention pour son utilisation dans le traitement de la dépendance à l'alcool ou le maintien du sevrage des alcooliques.

Un autre objet de l'invention concerne l'utilisation d'une micro-émulsion entre une phase aqueuse comprenant au moins un principe actif BCS (Biopharmaceutics Classification System) de classe III qui est le baclofène et une phase huileuse comprenant un excipient huileux auto-émulsifiable au contact de l'eau pour la fabrication d'un médicament destiné au traitement de la dépendance à l'alcool ou du maintien du sevrage des alcooliques.

*TABLEAU 2 : CARACTERISTIQUES DES DERIVES D'ACIDES GRAS COMMERCIALISES* (ABITEC, 2012)(GATTEFOSSE)(SASOL GERMANY GMBH, 2012) *NIC = NON CONNU*

| | Miglyol® 810 | Miglyol® 812 | Miglyol® 829 | Captex® 300 EP/NF | Captex® 355 EP/NF | Captex® 200P | Capmul® MCM, EP | Labrasol® | Gelucire® 50/13 |
|---|---|---|---|---|---|---|---|---|---|
| Composition | Triglycérides de : 65-80% Ac caprylique (C8:0) / 20-35% Ac caprique (C10:0) | Triglycérides de : 50-65% Ac caprylique/ 30-45% Ac caprique | Triglycérides de : 45-65% Ac caprylique/ 30-45% Ac caprique/ 15-20% Ac succinique | Triglycérides de : 50-80% Ac caprylique/ 20-50% Ac caprique | Triglycérides de : 50-80% Ac caprylique/ 20-50% Ac caprique | Propylène glycol de : 50-80% Ac caprique | 50-90% Ac caprylique/ 10-50% Ac caprique/ 45-75% mono; 20-50% di;10% triacylglycerols | Triglycérides et mono/di PEG-8 esters de : 50-80% Ac caprylique/ 20-50% Ac caprique | 40-50% Ac palmitique (C16)/ 48-58% Acstearique (C18) |
| Etat physique à Tamb | Liquide | Liquide | Liquide | Liquide | Liquide | Liquide | Semi-solide | Liquide | Semi-solide $T_f$= 50°C |
| Solubilités | Hexane, toluenediethylether, ethylacetate, acetone, isopropanol, ethanol 96% | Hexane, toluenediethylether, ethylacetate, acetone, isopropanol, ethanol 96% | Hexane, toluenediethylether, ethylacetate, acetone, isopropanol, ethanol 96% | Solvants organiques dont ethanol 95% | Solvants organiques dont ethanol 95% | N/C | N/C | Ethanol 96° : Très soluble Chloroforme, chlorure de méthylène : Très soluble Eau : Soluble **(HLB = 14)** Huiles minérales : Insoluble | Ethanol 96° : Insoluble Chloroforme : Facilement soluble Chlorure de méthylène : Facilement soluble Eau : Dispersible **(HLB = 13)** Huiles minérales : Insoluble |
| Indice de réfraction | 1,448-1,451 | 1,449-1,451 | 1,456-1,459 | 1,440-1,452 | 1,440-1,452 | N/C | N/C | 1,450-1,470 | N/C |
| Densité à 20°C (g/cm³) | 0,94-0,95 | 0,94-0,95 | 1,00-1,02 | 0,93-0,96 | 0,93-0,96 | N/C | N/C | 1,060-1,070 | N/C |
| Viscosité à 20°C (mPa.s) | 27-33 | 27-33 | 230-270 | 25-33 | 25-33 | N/C | N/C | 80-110 | N/C |
| Statut réglementaire | EP, USP, BP, GRAS | EP, USP, BP, GRAS | DMF, GRAS | EP/NF, DMF | EP/NF, DMF | EP/NF, USP, DMF | EP, DMF, GRAS | EP, USP/NF | EP, USP/NF |

La formulation pharmaceutique de l'invention peut être toute formulation pharmaceutique galénique orale connue de l'homme du métier, aussi bien liquide que solide.

Des formulations liquides appropriées sont toute formulation galénique liquide dans laquelle la microémulsion entre une phase aqueuse comprenant au moins un principe actif BCS (Biopharmaceutics Classification System) de classe III et une phase huileuse comprenant un excipient huileux auto-émulsifiable au contact de l'eau restera stable.

Les microémulsions, une fois formées, peuvent être directement introduites dans des gélules pour une administration orale, souples ou rigides, par exemple en gélatine.

De nombreuses techniques existent pour formuler une forme solide à partir d'une formule liquide *(Jannin, et al., 2008)*. La pulvérisation en chambre froide permet, par exemple, de congeler des gouttelettes qui recristallisent sous forme de particules sphériques solides.

La phase aqueuse d'une émulsion peut aussi être séchée par pulvérisation en lit d'air fluidisé pour former une « émulsion sèche ».

Celle-ci peut également être pulvérisée sur des sphères de neutres, ayant éventuellement des capacités d'adsorption, comme des sphères de cellulose microcristalline. Après séchage, ces sphères peuvent être utilisées pour formuler des comprimés avec des excipients de compression.

L'utilisation d'excipients solides à température ambiante mais liquides à chaud permet l'utilisation de la technique de thermogranulation ou d'extrusion-sphéronisation.

Enfin, l'adsorption sur support solide, telle que la cellulose microcristalline ou la silice, en mélangeur granulateur permet l'adsorption d'une grande quantité d'excipient liquide tout en maintenant de bonnes propriétés d'écoulement. Après séchage, le support imprégné de l'émulsion peut être utilisé pour formuler des comprimés avec des excipients de compression.

La formulation selon l'invention peut comprendre tout excipient additionnel classique de formulation.

La formulation selon l'invention comprend de 10 à 80 mg de principe actif par unité de dose, de préférence de 20 à 60.

## EXEMPLES

### Exemple 1 : MATERIELS ET METHODES

#### a. Matières premières

##### i. Principe actif

Le Baclofène provient de la société PCAS.

##### ii. Promoteurs d'adsorption

Le Labrasol® et la Gélucire® 50/13 proviennent de chez Gattefossé®, le Miglyol® 810 de chez Sasol® Germany GmBH et le Capmul® MCM et le Captex® 355 de la société Abitec®.

##### iii. Autres excipients

Les excipients utilisés au cours des différents essais sont les suivants : Acide fumarique (Merck), Avicel® PH102 (cellulose microcristalline, FMC Biopolymer), Neusilin® (aluminométasilicate de magnésium, FugiChemicalIndustry), HPMC 603 (hydroxypropylméthylcellulose, Shin Etsu), Tween® 80 (polysorbate 80, Sigma).

#### b. Essais de solubilité du baclofène

Au préalable, il est important de déterminer les solubilités du principe actif dans les excipients que l'on veut évaluer. En effet, en vue des essais sur cellules, il est important que le principe actif se présente sous forme moléculaire afin d'en obtenir le passage membranaire. Les solubilités sont déterminées par une méthode visuelle, par introduction de quantités exactement pesées de baclofène dans l'excipent sélectionné, après agitation pendant 24h. Dans un premier temps, les solubilités, à température ambiante, du baclofène dans le Labrasol®, le Miglyol® 810 et le Captex® 355 sont évaluées. La solubilité du baclofène dans la Gélucire® 50/13 est déterminée après fonte de l'excipient à une température supérieure à 50°C puis placé dans une étuve thermostatée à 60°C. Dans un second temps, afin d'augmenter la solubilité du baclofène en milieu aqueux, celle-ci est évaluée dans une solution acidifiée d'acide fumarique 0,01 M. L'acide fumarique n'induisant pas de problème de stabilité avec le baclofène selon un rapport interne de compatibilités binaires.

### c. Formulations lipidiques

#### i. Microémulsions

Le Miglyol® 810, le Capmul® MCM et le Captex® 355 sont des excipients non miscibles à l'eau. Le baclofène étant une molécule hydrophile, il est choisi de formuler des microémulsions à partir de ces excipients par l'utilisation de diagrammes pseudo-ternaires. C'est-à-dire ayant un ratio surfactant-cosurfactant de composition fixe mais en faisant varier la proportion de ce mélange avec les phases huileuse et aqueuse. L'objectif est de formuler une microémulsion huile-dans-eau (H/E) et une microémulsion eau-dans-huile (E/H).

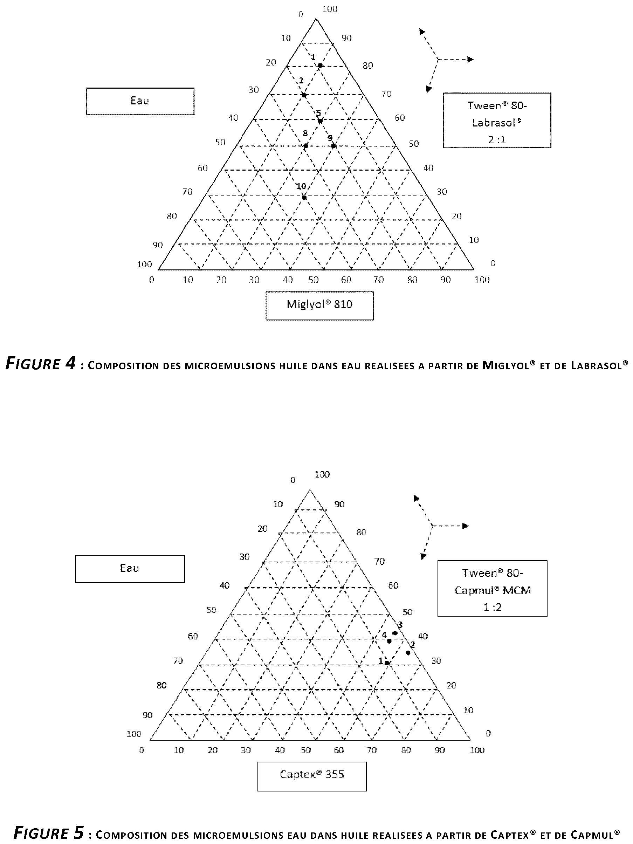

Six essais sont réalisés pour l'obtention d'une microémulsion H/E utilisant un mélange surfactant-cosurfactant Tween®80-Labrasol® en proportion 2:1 et le Miglyol®810 pour la phase huileuse (figure 4).

Quatre essais sont réalisés pour l'obtention d'une microémulsion E/H utilisant un mélange surfactant-cosurfactant Tween®80-Capmul®MCM en proportion 1:2 et le Captex®355 pour la phase huileuse (figure 5).

La phase aqueuse, composée d'eau purifiée, est ajoutée en dernier sous agitation. Les microémulsions se forment alors instantanément. Puis, une fois la composition des mélanges sélectionnée, des essais sont réalisés avec une solution aqueuse d'acide fumarique 0,01 M à 6 mg/ml de baclofène.

#### ii. Mélanges binaires

Le Labrasol® et la Gélucire® 50/13 sont des excipients s'auto-émulsionnant en présence d'eau. Divers ratio eau/Labrasol® ou eau/Gélucire® 50/13 sont testés. Les mélanges eau/Gélucire® 50/13 sont réalisés à une température supérieure à 50°C tandis que les mélanges eau/Labrasol® sont réalisés à température ambiante.

#### iii. Caractérisation

##### 1. Viscosités

La viscosité des émulsions est déterminée à l'aide d'un viscosimètre rotatif Brookfield DV-II+ et du mobile SC4-18 utilisant une géométrie de Couette cylindrique.

##### 2. Granulométries

La granulométrie des émulsions est mesurée par diffusion quasi-élastique de la lumière sur un granulomètre Coulter® N4 plus. Les mesures sont réalisées à un angle de 90° directement sur les émulsions en tenant compte des caractéristiques du milieu dispersant (viscosité, indice de réfraction) puis après dilution dans l'eau. Le facteur de dilution est dépendant de l'intensité mesurée à 90° qui doit être comprise entre $5.10^4$ et $1.10^6$ coups/sec pour la validité de la mesure. Chaque mesure est réalisée en triplicat.

##### 3. Stabilités

La stabilité des émulsions est déterminée visuellement sur deux mois par l'observation de l'apparition ou non d'un déphasage et par mesure granulométrique. Les mesures obtenues à un temps t=14 jours et t=45 jours sont comparées.

### d. Sélection des formules : Essais de perméabilité sur cellules Caco-2

#### i. Culture cellulaire

Les cellules Caco-2 ont été cultivées et se sont différenciées pendant 21 à 30 jours. Elles ont subi un nombre de repiquage inférieur à 110. Le milieu de culture est un milieu DMEM (Dulbecco'sModifiedEagle Médium) supplémenté de 10 % de sérum foetal de veau inactivé, d'1 % d'acides aminés non-essentiels et d'antibiotiques.

#### ii. Test de perméabilité

Les cellules Caco-2 ont permis l'évaluation du potentiel de huit formules, présentées dans les tableaux 5a, 5b, 5c et 5d, dans l'amélioration de la perméabilité apparente du baclofène. Les dilutions effectuées sont au choix du sous-traitant en fonction de la concentration en baclofène mesurable par la technique de dosage et en fonction de concentration la plus faible non toxique donnée par des études de toxicité préliminaires. Les compositions des milieux apicaux et basolatéraux sont données dans le tableau 3 ci-dessous.

*Tableau 3 : Composition des milieux apicaux et basolatéraux des essais sur cellules Caco-2*

| Milieu apical | Milieu basolatéral |
|---|---|
| HBSS | HBSS |
| 10 mM HEPES | 10 mM HEPES |
| 0,1% BSA | 0,1% BSA |
| 200 $\mu$M Lucifer Yellow | - |
| pH 6,8 | pH 7,4 |

Des prélèvements sont réalisés après l'introduction de l'échantillon dans le milieu apical : à T5 minutes et T125 minutes dans le compartiment donneur et à T65 minutes et T125 minutes dans le compartiment receveur. Le dosage du baclofène est réalisé par une méthode d'analyse LC/MS/MS. La perméabilité apparente du baclofène et le rapport de recouvrement sont alors calculés de la façon suivante :

-

$$\text{Papp} = [\Delta Q/(\Delta t \times A)] / C_0 \text{ où Papp = perméabilité apparente } (\text{cm.s}^{-1})$$

$\Delta Q$ = la différence de la quantité de baclofène mesurée entre les deux prélèvements $\Delta t$ = l'intervalle de temps entre les deux prélèvements
A = l'aire d'exposition (cm$^2$)
$C_0$ = la concentration initiale déposée dans le compartiment donneur.

Le résultat est interprété de la façon suivante :

*Tableau 4 : Interprétation de résultats de perméabilité apparente*

| Perméabilité | Faible | Intermédiaire | Haute |
|---|---|---|---|
| $P_{app}$ x $10^{-6}$ cm/s | < 0,5 | 0,5 à 5 | > 5 |

Tableau 5a : Composition centésimale microémulsion huile-dans-eau

| Ingrédients | Pourcentage |
|---|---|
| Captex 355 | 60 |
| Capmul MCM | 20 |
| Tween 80 | 10 |
| Solution aqueuse de Baclofène | 10 |
| **Total** | **100** |

Tableau 5b : Composition centésimale de l'émulsion huile-dans-eau

| Ingrédients | Pourcentage |
|---|---|
| Miglyol 810 | 20 |
| Labrasol | 20 |
| Tween 80 | 40 |
| Solution aqueuse de Baclofène | 20 |
| **Total** | **100** |

Tableau 5c : Composition centésimale de l'émulsion de la solution eau-labrasol

| Ingrédients | Pourcentage (solution à 25% labrasol) | Pourcentage (solution à 10% labrasol) | Pourcentage (solution à 5% labrasol) | Pourcentage (solution à 1% labrasol) |
|---|---|---|---|---|
| Labrasol | 25 | 10 | 5 | 1 |
| Solution aqueuse de Baclofène | 75 | 90 | 95 | 99 |
| **Total** | **100** | **100** | **100** | **100** |

Tableau 5d : l'émulsion de la solution eau-gélucire

| Ingrédients | Pourcentage (solution à 5% gélucire) | Pourcentage (solution à 1% gélucire) |
|---|---|---|
| Gélucire 50/13 | 5 | 1 |
| Solution aqueuse de Baclofène | 95 | 99 |
| **Total** | **100** | **100** |

- Recouvrement =

$$\left[ \text{Quantité résiduelle dans le compartiment donneur à T125} + \text{Quantité accumulée dans le compartiment receveur entre T5 et T125} \right] / \text{Quantité à T5 dans le compartiment donneur}$$

Le résultat doit être compris entre 75 et 125 %.

Chaque essai est réalisé trois fois. Pour la validité de l'essai, la perméabilité d'un composé de référence est aussi mesurée en tant que contrôle positif. Il s'agit du métoprolol à 20 µM, une molécule empruntant un transport actif transcellulaire. Le baclofène seul est aussi utilisé en tant que contrôle négatif.

*iii. Evaluation de l'intégrité cellulaire*

1. Mesure de la résistance transépithéliale

Avant chaque essai, une mesure de la résistance transépithéliale est réalisée. Celle-ci doit être supérieure à 1500Ω. Une seconde mesure est réalisée après le test. Si la perte de résistance est supérieure à 30 %, il est conclu à une altération de la monocouche.

2. Test à la Lucifer Yellow

Au cours des essais, le passage du compartiment apical vers basolatéral de la Lucifer Yellow, un marqueur de passage paracellulaire, est également évalué. 200 µM de Lucifer Yellow est co-incubé avec le composé test et sa perméabilité est évaluée de la même façon que pour ce dernier. Les concentrations sont mesurées par fluorescence dans les deux compartiments au début et à la fin de l'essai.

3. Test MTS-PMSLe test MTS-PMS est un dérivé du test MTT, ce test permet de vérifier la viabilité des cellules. Les cellules vivantes transforment le MTS en formazan soluble dans le milieu de culture. La quantité de formazan produit est évaluée par la mesure de l'absorbance à 490 nm. Cette quantité est directement reliée à la proportion de cellules vivantes dans le milieu.

**e. Mise sous forme solide : Technique d'adsorption/granulation**

Suite aux résultats des essais sur cellules, le Labrasol® est l'excipient sélectionné pour la formulation d'une forme solide. Les essais d'adsorption/granulation se font dans un mélangeur/granulateur Diosna PVAC 10. Une cuve de 2L et une buse de pulvérisation de 0,8 mm sont utilisés.

i. Essais de granulation

Des essais de granulation sont réalisés sur cellulose microcristalline (Avicel® PH102) selon le tableau 6. La vitesse de rotation de la pâle de fond est de 200 rpm et celle de l'émotteur de 1500 rpm. Le débit de pulvérisation est de 30 g/min. Un temps de séchage d'une heure à 50°C sous vide est nécessaire pour l'obtention d'un grain contenant une humidité résiduelle inférieure à 5 % (thermobalanceMettler). Le grain est ensuite tamisé sur mailles de 0,5 mm.

*Tableau 6 : Composition de l'essai de granulation*

| Charge sèche | Fonctionnalité des excipients | Formule (%) | Quantité (g) QSP 300 g |
|---|---|---|---|
| Avicel® pH 102 | Adsorbant | 95 | 285 |
| HPMC 603 | Liant | 5 | 15 |
| **Solution de mouillage** | - | **Formule (%)** | **Quantité (g) QSP 300 g** |
| Eau purifiée | Agent de granulation | 50 | 150 |
| Labrasol® | Promoteur d'absorption | 50 | 150 |

La granulométrie est mesurée par granulomètre laser Malvern® et les données sont traitées à l'aide du logiciel Mastersizer 2000. Les aptitudes du grain à la compressibilité et à l'écoulement sont évaluées par mesure du volume apparent sous tassement (voluménomètre STAV 2003) et calcul de l'indice de Carr ainsi que par mesure du temps d'écoulement sur 100 g de poudre à travers un entonnoir normalisé. Ces essais sont réalisés selon la Pharmacopée Européenne en vigueur *(Pharmacopée Européenne 7ème édition, 2012)*.

ii. Essais d'adsorption

Des essais d'adsorption simple sont également réalisés en mélangeur par introduction de Labrasol® dans l'Avicel® PH102 ou le Neusilin® (Magnesiumaluminometasilicate) sous agitation des pâles respectivement à 150 et 300 rpm. Le débit est de 20 g/min et les essais se font à chaud à 40°C sous vide afin de fluidiser l'excipient huileux le Labrasol. Les adsorbants suivants sont également testés : Fujicalin®, Hubersorb®, 600 et Syloïd ®244FP.

**Exemple 2 : RESULTATS**

**a. Solubilités du baclofène**

Les solubilités indiquées dans le tableau 7 ci-dessous sont déterminées en fonction de l'observation visuelle de particules dans les milieux.

*Tableau 7: Résultats de solubilité du baclofène dans divers milieux*

| Produit | Température | Solubilités du baclofène |
|---|---|---|
| Miglyol®810 | Ambiante | < 0,1 mg/ml |
| Captex® 355 | Ambiante | < 0,1 mg/ml |
| Labrasol® | Ambiante | 0,2-0,4 mg/ml |
| Gélucire® 50/13 | 60°C | 0,5-1 mg/ml |
| Milieu aqueux Acide fumarique 0,01M | Ambiante | 6-7 mg/ml |

Le baclofène apparaît soluble en milieu aqueux et pratiquement insoluble dans les excipients huileux.

**b. Formulations lipidiques**

*i. Microémulsions*

En fonction des compositions réalisées, différentes observations peuvent être faites. Concernant les microémulsions à base de Miglyol® 810 et de Labrasol® (figures 4 et 5), les compositions 1, 2 et 3 permettent l'obtention de milieux limpides et la composition 10 produit un milieu laiteux. Les compositions 8 et 9 sont opaques et un déphasage est obtenu après 24h.

Les quatre compositions réalisées à base de Captex® 355 et Capmul® MCM fournissent des microémulsions claires.

Les microémulsions réalisées avec principe actif ne présentent pas de différence visuelle par comparaison avec celles sans principe actif.

*ii. Mélanges binaires*

Le Labrasol® est miscible en toutes proportions dans l'eau. Cependant, un mélange clair et homogène apparaît pour des concentrations en Labrasol supérieures à 1 % dans l'eau. Pour des concentrations inférieures à 1 %, le milieu se trouble et les particules visibles à l'oeil nu sédimentent.

La Gélucire® est également miscible à l'eau mais seuls des mélanges eau/Gélucire® à des concentrations inférieures à 5 % restent sous forme liquide à température ambiante.

*iii. Caractérisation*

En fonction de critères discutés par la suite, certaines compositions sont retenues pour essais de perméabilités sur cellules et sont ici caractérisées.

*1. Viscosités*

Les viscosités des microémulsions sont données dans le tableau 8 ci-dessous. La valeur du couple fournit une indication sur la fiabilité de la mesure.

*Tableau 8 : viscosité des microémulsions à température ambiante*

| Echantillon | Vitesse de rotation du mobile (rpm) | Valeur du Couple (%) | Viscosité mesurée (cP) |
|---|---|---|---|
| Microémulsion Miglyol®/ Labrasol® | 6 | 85 | 423 |
| Microémulsion Capmul®/ Captex® | 30 | 60 | 59 |

Les viscosités des mélanges binaires sont proches de celle de l'eau.

*2. Granulométries*

Les granulométries sont mesurées 14 jours après la préparation des systèmes, avant et après dilution pour les microémulsions ainsi que pour les mélanges binaires Eau/Labrasol®. Les résultats sont indiqués dans les tableaux 9 et 10.

Pour les microémulsions, la dilution provoque une déstabilisation et l'apparition d'un milieu opaque dont l'intensité à 90° est trop élevée pour la mesure de tailles des particules. Une dilution est donc nécessaire pour avoir une intensité comprise dans l'intervalle recommandé par le fabricant du granulomètre. Les caractéristiques et notamment la viscosité de l'émulsion Miglyol®/Labrasol® non diluée ne permettent pas de mesures sur le granulomètre Coulter.

Pour les mélanges Eau/Labrasol®, la dilution réalisée est identique à celle des essais sur cellules Caco-2.

*3. Stabilités des microémulsions à T = 45jours*

Des mesures granulométriques, indiquées dans le tableau 9, sont également effectuées et sont comparées à celles obtenues à 14 jours. Aucun déphasage n'apparait pour les microémulsions placées à température ambiante après 45 jours.

**c.** **Essais de perméabilité sur Caco-2 et intégrités membranaires**

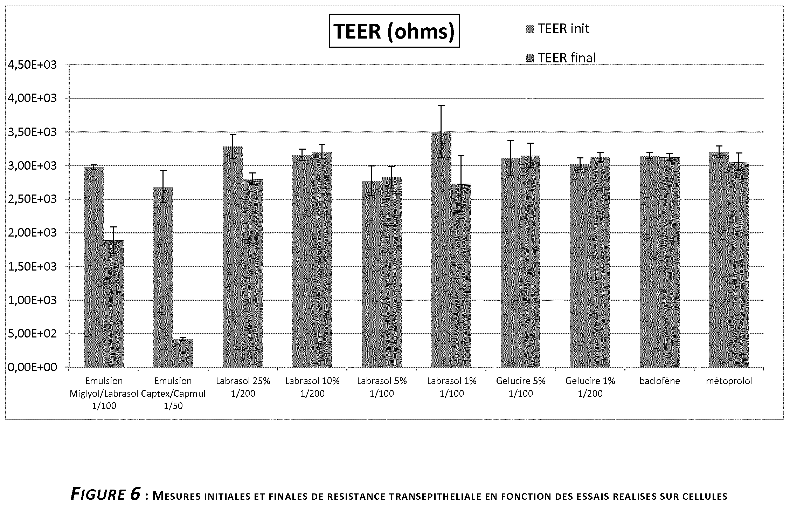

La mesure de la résistance transépithéliale (figure 6) avant et après l'essai de perméabilité nous informe sur le potentiel toxique des huit formules ainsi que des différents témoins. Seules les microémulsions induisent une variation de résistance supérieure à 30 % entre les mesures initiales et finales indiquant une perte de l'intégrité membranaire. Les 6 autres formules ainsi que les témoins provoquent une variation inférieure à 30 % indiquant une récupération de la résistance des cellules après l'essai. L'intégrité membranaire n'est donc pas affectée par ces formules.

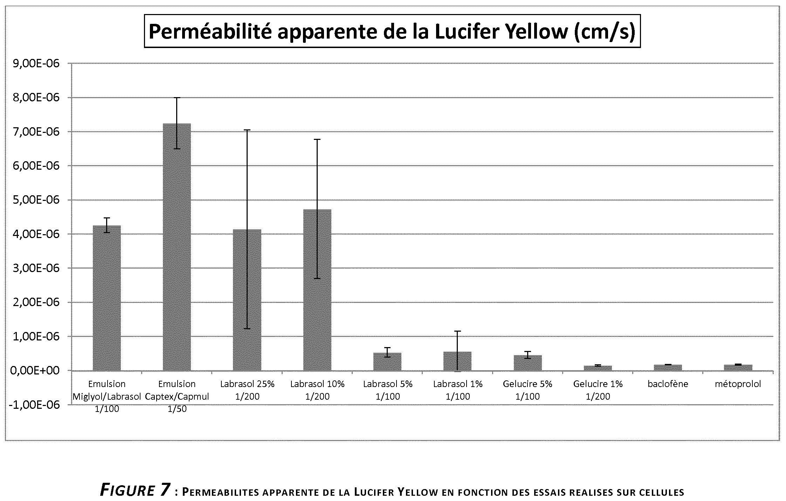

Concernant une action des formules sur les jonctions serrées du tapis cellulaire, la perméabilité apparente de la Lucifer Yellow est suivie pour chaque essai (figure 7). On observe une forte action des microémulsions et des formules de Labrasol à 10 et 25 % sur le passage paracellulaire entre 4 et $7.10^{-6}$ cm/s. Cependant, pour les microémulsions, ce résultat peut être relié à la déstabilisation membranaire. Les formules de Labrasol® à 1 et 5 % ainsi que celles de Gelucire® 5 % provoquent également un passage de la Lucifer Yellow supérieur à $3.10^{-7}$ cm/s. Mais celui-ci reste faible car inférieur à $0,5.10^{-6}$ cm/s (tableau 4). Les témoins, quant à eux, ne provoquent pas d'ouverture des jonctions serrées.

Le test MTS-PMS montre que nos formules ne génèrent pas de toxicité cellulaire.

*TABLEAU 9 : GRANULOMETRIES DES MICROEMULSIONS A 20°C A T=14J ET T=45J*

| Mesures à T=14 jours | | | | | |
|---|---|---|---|---|---|
| | | Dilution | Milieu dispersant | Proportion (%) | Taille (nm) |
| Emulsions **placebo non diluée** | Emulsion Miglyol®/ Labrasol® | N/A | Viscosité =423 cP, Indice de réfraction = 1,45 | Absence de résultats | |
| | Emulsion Captex®/ Capmul® | N/A | Viscosité=59 cP, Indice de réfraction= 1,45 | 100 | 21,3 |
| Emulsions avec **actif non diluées** | Emulsion Miglyol®/ Labrasol® | N/A | Viscosité =423 cP, Indice de réfraction = 1,45 | Absence de résultats | |
| | Emulsion Captex®/ Capmul® | N/A | Viscosité=59 cP, Indice de réfraction= 1,45 | 100 | 24,2 |
| Emulsions **placebo après dilution** | Emulsion Miglyol®/ Labrasol® | 1/5000 | Eau | 12-17 | 1 |
| | | | | 10-20 | 50-70 |
| | | | | 25-80 | 130-195 |
| | | | | 50-60 | 218-285 |
| | Emulsion Captex®/ Capmul® | 1/10000 | Eau | 1-2 | 1-3 |
| | | | | 98-99 | 230-500 |
| Emulsions **avec Actif après dilution** | Emulsion Captex®/ Capmul® | 1/10000 | Eau | 5 | 17 |
| | | | | 11-12 | 75-77 |
| | | | | 88 | 1000 |
| | Emulsion Miglyol®/ Labrasol® | 1/5000 | Eau | 100 | 180-390 |

(suite)

| Mesures à T=45 jours | | | | | |
|---|---|---|---|---|---|
| Emulsions **avec actif non diluées** | Emulsion Miglyol®/ Labrasol® | N/A | Viscosité =423 cP, Indice de réfraction = 1,45 | Absence de résultats | |
| | Emulsion Captex®/ Capmul® | N/A | Viscosité=59 cP, Indice de réfraction= 1,45 | 100 | 27,2 |
| Emulsions **avec Actif après dilution** | Emulsion Miglyol®/ Labrasol® | 1/5000 | Eau | 12-13 | 1 |
| | | | | 14-20 | 70-74 |
| | | | | 65-87 | 190-200 |
| | Emulsion Captex®/ Capmul® | 1/10000 | Eau | 1 | 4-5 |
| | | | | 99 | 530-630 |

### TABLEAU 10 : GRANULOMETRIE DES MELANGES EAU/LABRASOL A 20°C A T=7J

| Labrasol® 25 % | Labrasol® 10 % | Labrasol® 5 % | Labrasol® 5 %**Placebo** | Labrasol® à 1 % |
|---|---|---|---|---|
| 50% à 1 nm | 50% à 1 nm | 30% à 1 nm | 20% à 1 nm | > 2 $\mu$m |
| 50% à 15 nm | 50% à 12 nm | 40-50% à 10 nm | 80% à 13,5 nm | |
| | | 20-30% à 400 nm | | |
| | Labrasol® 10% - 1/200 | Labrasol® 5% - 1/100 | | |
| | 525,7 nm +/- 105,6; IP= 0,044 | 657,5 nm +/- 170; IP=- 0,091 | | |

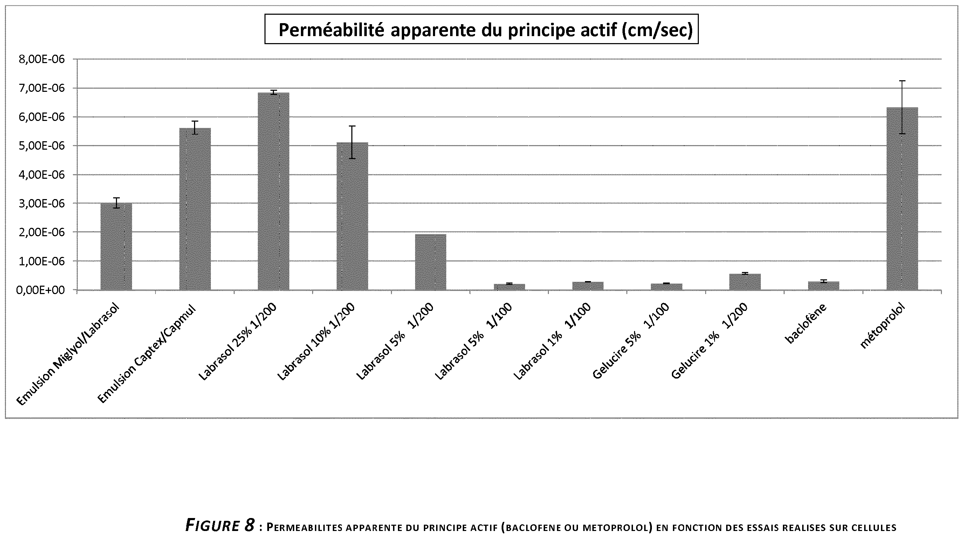

Les résultats de perméabilité apparente du baclofène en fonction des différentes formules sont donnés dans la figure 8.

Les essais sur cellules confirment la faible perméabilité du baclofène avec une perméabilité apparente inférieure à $3.10^{-7}$ cm/s. Cette perméabilité est augmentée à $3.10^{-6}$ cm/s dans le cas de l'émulsion Miglyol®/Labrasol®. Elle devient très haute pour les formules Captex®/Capmul® et Labrasol® à 25 et 10 % allant de 5 à $7.10^{-6}$ cm/s. Cependant, de même que pour les résultats concernant la Lucifer Yellow, les perméabilités associées aux microémulsions sont certainement dues à la perte d'intégrité membranaire observée. Les formules de Labrasol® à 1 et 5 % ainsi que les formules contenant de la Gélucire® ne permettent pas d'augmentation de la perméabilité apparente du baclofène. La perméabilité apparente du métoprolol est très haute, car supérieure à $6.10^{-6}$ cm/s, comme attendu. Celui-ci est un témoin du passage transcellulaire et son absence d'effet sur l'ouverture des jonctions serrées démontrée ci-dessus valide les conditions expérimentales. Ainsi, les formules les plus prometteuses pour l'augmentation du passage membranaire du baclofène sont les formules à base de Labrasol®, plus particulièrement celles à 10 % et 25 % qui permettent respectivement des augmentations de 20 et 28 fois la perméabilité apparente du baclofène non formulé. Par ailleurs, les taux de recouvrement calculés au cours des essais sont aux alentours de 100 % ce qui confirme l'absence de dégradation du produit pendant l'essai. Aucune perte n'est observée.

**d. Passage sous forme solide**

*i. Granulation*

Un grain contenant 33 % de Labrasol® est obtenu. Celui-ci est de couleur jaune et d'aspect gras au toucher.

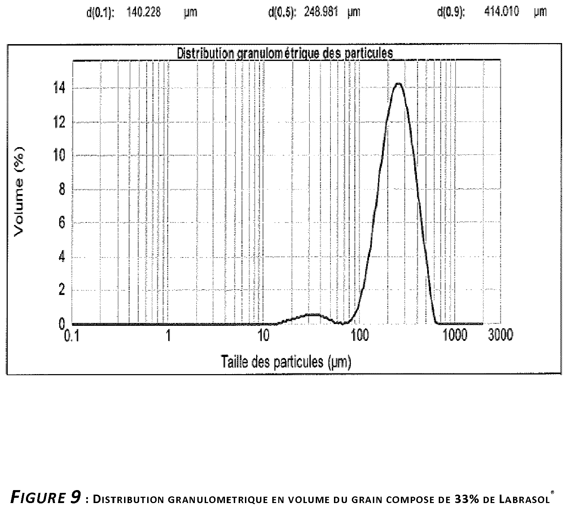

Les résultats de granulométrie sont indiqués dans la figure 9.

Les données obtenues par le voluménomètre et le temps d'écoulement sont indiqués dans le tableau 11 ci-dessous.

**Tableau 11 : Volumes après tassement, densités, Indice de Carr et temps d'écoulement du grain contenant 33 % de Labrasol®**

| | Pesée (g) | V0 (ml) | V10 (ml) | V500 (ml) | V1250 (ml) | $\rho$0 (g/ml) | $\rho$ tassée (g/ml) | V10-V500 (ml) | Indice de Carr (100x (V10-V1250) /V10) | Temps d'écoulement (s) |
|---|---|---|---|---|---|---|---|---|---|---|
| Grain 33% Labrasol® | 100,14 | 184 | 172 | 158 | 158 | 0,54 | 0,63 | 14 | 8,14 | |

*ii. Adsorption*

Les essais d'adsorption sur cellulose microcristalline à la même concentration que celle obtenue en granulation fournissent une poudre jaune, grasse d'aspect « sable mouillé » et formant des amas.

Les essais d'adsorption sur Neusilin® permettent l'obtention d'une poudre contenant 60 % de *Labrasol®.* Ce mélange est blanc, d'aspect sec et très volatil.

L'objectif de cette étape est de choisir un excipient en vue de futurs essais de compression, les excipients chargés sont caractérisés en écoulement par la mesure du débit d'écoulement et de l'indice de Carr (Ic) (Tableau 12).

**TABLEAU 12 : CARACTERISATION DE L'ECOULEMENT DES EXCIPIENTS CHARGES ET NON CHARGES EN LABRASOL®**

| | Débit d'écoulement (g/s) | Densité (g/mL) | Ic (%) | Coulabilité selon Ic |
|---|---|---|---|---|
| Avicel® PH102 | 0 | 0,34 | 15,1 | Bon |
| Avicel® PH102 + Labrasol® | 0 | 0,34 | 16,4 | Assez bon |
| Fujicalin® | 20,2 | 0,47 | 6,9 | Excellent |
| Fujicalin® + Labrasol® | 19,3 | 0,65 | 9,5 | Excellent |
| Neusilin® US2 | 2,9 | 0,15 | 8,9 | Excellent |
| Neusilin® US2 + Labrasol® | 23 | 0,48 | 6,0 | Excellent |
| Neusilin® UFL2 | 0 | 0,11 | 25,0 | Passable |
| Neusilin® UFL2 + Labrasol® | 0 | 0,21 | 29,5 | Mauvais |
| Hubersorb® 600 | 0 | 0,08 | 25,0 | Passable |
| Hubersorb® 600 + Labrasol® | 0 | 0,17 | 32,9 | Très mauvais |
| Syloïd® 244FP | 0 | 0,07 | 14,8 | Bon |
| Syloïd® 244FP + Labrasol® | 0 | 0,30 | 12,5 | Bon |

Ainsi, les poudres adsorbées sans écoulement (Hubersorb® 600, Avicel® PH102, Neusilin® US2, Syloïd® 244FP) ne sont pas retenues. Le Neusilin® US2 et le Fujicalin® adsorbés présentent quant à eux un très bon écoulement et pourront donc être utilisés.

## I. DISCUSSION

### Choix des formules pour essais de perméabilité et stabilité

Comme attendu, la solubilité du principe actif dans les promoteurs d'absorption liquide s'est avérée très faible car inférieure à 1 mg/ml. En effet, le baclofène est un PA hydrophile, comme l'indique son log P de - 0,96 *(Benet, et al., 2011),* alors que les promoteurs d'absorption sont des huiles.

Cependant, les produits tels que le Labrasol® et la Gélucire® 50/13 ont la particularité de présenter un HLB élevé de l'ordre de 13-14 (Gattefossé) qui permet leur miscibilité à l'eau. Ainsi, il a été décidé de réaliser des mélanges binaires de Labrasol® ou de Gélucire® avec une solution aqueuse contenant le baclofène préalablement solubilisé. Les ratios Labrasol®/eau sont choisis afin d'évaluer un impact éventuel de la concentration en Labrasol® sur les cellules. Concernant la Gélucire®, on cherche à ce que le mélange reste sous forme liquide à température ambiante, ce qui limite la gamme des concentrations inférieures à 5 %. Il est observé qu'aux faibles concentrations (inférieures à 5 % pour la Gélucire® et inférieure à 1 % pour le Labrasol®) les mélanges de Labrasol et de Gélucire présentent un trouble. La mesure granulométrique indique que les particules du mélange de Labrasol® sont très supérieures à celles observées pour les concentrations plus élevées (2 $\mu$m versus 13 nm). Les tailles obtenues pour les solutions les plus concentrées peuvent expliquer la sédimentation observée. Finalement, quatre formules de Labrasol® à 1, 5, 10 et 25 % ainsi que deux formules de Gélucire® à 1 et 5 % ont été préparées et envoyées au sous-traitant.

En ce qui concerne les microémulsions composées de Miglyol®/Labrasol® fournissent trois compositions claires et stables visuellement (tableau 13).

### TABLEAU 13 : MICROEMULSIONS MIGLYOL®/LABRASOL® STABLES

| Microémulsion | Miglyol® | Labrasol® | Tween® 80 | Phase aqueuse |
|---|---|---|---|---|
| 1 | 10 % | 27 % | 53 % | 10 % |
| 2 | 10 % | 23 % | 47 % | 20 % |
| 3 | 20 % | 20 % | 40 % | 20 % |

Pour essai sur cellules, la composition 3 est retenue en raison de divers critères. D'une part, le Tween® 80 utilisé en tant que tensioactif est connu pour ses problèmes de toxicité, la formule qui en contient le moins semble donc plus sûre. De plus, la proportion en eau est plus importante et permet donc l'introduction de davantage de principe actif. Enfin, la quantité de Miglyol® importante d'un point de vue de la promotion d'absorption est aussi plus importante.

Les quatre microémulsions préparées à base de Captex®/Capmul® produisent des formules claires et stables (tableau 14).

### TABLEAU 14 : MICROEMULSIONS CAPTEX®/CAPMUL® STABLES

| Microémulsion | Captex® | Capmul® | Tween® 80 | Phase aqueuse |
|---|---|---|---|---|
| 1 | 60 % | 20 % | 10 % | 10 % |
| 2 | 64 % | 22 % | 11 % | 3 % |
| 3 | 55 % | 28 % | 14 % | 3 % |
| 4 | 56 % | 26 % | 13 % | 5 % |

En raison des mêmes critères que précédemment, la composition 1 est sélectionnée pour essai sur cellules.

Ainsi, la perméabilité du baclofène incorporé dans deux microémulsions de compositions différentes est mesurée.

Le mécanisme de passage du baclofène *(Merino, et al.,* 1989) consiste en un passage actif grâce à des transporteurs mais ceux-ci sont saturés à forte dose. Ne connaissant pas la composition des cellules Caco-2 en transporteurs, une concentration élevée en baclofène dans les formules est recherchée de sorte que les transporteurs présents soient saturés. Ainsi, seule l'augmentation du passage paracellulaire sera évaluée. La concentration à saturation du baclofène dans l'eau est de 3 mg/ml *(rapport interne de préformulation).* Cependant, sa solubilité est fortement dépendante du pH comme les fonctions amines et carboxyliques présentent sur la molécule l'indiquent. Ainsi l'ajout d'acide fumarique à 0,01 M, démontré comme compatible avec le baclofène par le rapport interne de préformulation, permet de doubler la solubilité du baclofène à 6 mg/ml. Le pH est alors de 3,88.

Selon *Constantinides et al (1996),* le pH d'une microémulsion n'influence pas sa stabilité ni sa taille de particules si les tensioactifs utilisés ne sont pas ionisés ou ionisables. Ainsi, le remplacement de la phase aqueuse des microémulsions par l'acide fumarique 0,01 M à 6 mg/ml de baclofène ne provoque pas de déstabilisation visuelle des microémulsions.

La stabilité des systèmes est aussi suivie par mesures granulométriques 14 et 45 jours après la réalisation des microémulsions.

Concernant l'émulsion Captex®/Capmul®, une mesure de 21,3 nm est obtenue à 14 jours pour la formule placebo, ce qui confirme la présence d'une microémulsion. Une mesure à 24,2 nm est obtenue pour la formule avec principe actif indiquant que le principe actif n'influence pas la taille des particules comme le suggère l'étude de *(Prajapati, et al., 2012)*. Cependant, les études de *Buyukozturk et al (2010)* et *Gundogdu et al (2011)* font apparaître une influence du principe actif sur la taille des globules. Après 45 jours, la mesure est de 27,2 nm, ce qui confirme la stabilité du système. Afin d'étudier l'influence de la dilution sur notre échantillon, différentes dilutions ont été réalisées. Au cours de cette étude, les systèmes deviennent opaques indiquant une déstabilisation des formules et une possible inversion de phase comme l'explique *Prajapati et al.* L'inversion de phase est confirmée par les mesures sur l'émulsion Captex®/Capmul® puisque les mesures de taille apparaissent largement supérieures, de l'ordre de 230 à 500 nm. La présence de l'actif ne semble ici non plus avoir d'incidence sur ces mesures.

La mesure de granulométrie de l'émulsion Miglyol®/Labrasol® n'a pas pu être réalisée à cause de la viscosité élevée du milieu mesurée à 423 cP. Pour évaluer la stabilité de l'émulsion Miglyol®/Labrasol®, le système est dilué en pensant que la dilution n'affecte pas la taille des particules de l'émulsion, effet soutenu par *Prajapati et al.* Pour l'émulsion Miglyol®/Labrasol®, les mesures avec actif à 14 jours semblent supérieures à celles obtenues sur placebo. Cependant, les mesures à 45 jours avec actif sont du même ordre de grandeur que celles de l'émulsion placebo. Il semble donc difficile ici de conclure sur la stabilité granulométrique de l'émulsion Captex®/Capmul®. Nous pouvons tout de même observer visuellement l'absence de déphasage au cours des deux mois d'observation, ce qui permet de conclure sur la stabilité macroscopique des systèmes.

### a. Interprétation des résultats de perméabilité mis en relation avec la caractérisation des formules

L'observation d'une très faible perméabilité du baclofène non formulé, inférieure à $0,5.10^{-6}$ cm/s, permet de supposer que la concentration appliquée (30 $\mu$g/ml) est proche de la saturation des transporteurs. Cette valeur peut être comparée à celles des essais Labrasol® 25 %, 10 % et Gélucire® 1% dilués de la même façon 200 fois. La Gélucire® à 1 % ne permet pas d'augmentation de la perméabilité apparente tandis que les essais avec le Labrasol® permettent une forte augmentation de cette perméabilité par 20 et 28 fois.

En ce qui concerne les formules diluées 100 fois (Labrasol® 5 %, Labrasol® 1 % et Gélucire® 5 %), les valeurs de perméabilité sont très faibles, parfois même inférieures à celle du baclofène non formulé. Or, les concentrations en baclofène déposé sur cellules pour ces essais sont deux fois plus importantes que celle du témoin négatif. On peut donc penser que la concentration du témoin ne se situe pas tout à fait à la saturation complète des transporteurs. Ainsi, les perméabilités ne peuvent pas totalement être comparées entre-elles puisque celles-ci dépendent de la concentration en principe actif qui s'avère différente d'une formule à l'autre. Les essais de Gélucire® illustrent bien ce propos. En effet, la concentration en Gélucire® est plus importante pour la Gélucire® 5 % que pour la Gélucire® 1 %, même après dilution. Ceci pourrait expliquer les résultats de perméabilité de la Lucifer Yellow qui sont plus importants dans le premier cas et laisser penser à un léger effet de la Gélucire® sur le passage paracellulaire. Cependant, la perméabilité du baclofène est plus importante pour la Gélucire® 1 %, certainement en raison de la concentration en baclofène plus faible qui permet un passage facilité de la molécule.

Malgré l'effet de la concentration en baclofène, on peut tout de même observer une forte différence de perméabilité entre les formules Labrasol® 10 et 25 % et les formules Labrasol® 1 et 5 %. Dans un premier temps, la recherche de différences de structure des mélanges est recherchée notamment par la mesure granulométrique des particules. En effet, *Koga et al,* ont démontré l'importance de la concentration en Labrasol® sur la taille des particules qui augmenterait avec la concentration. Le baclofène serait alors mieux entouré par le Labrasol® ce qui permettrait d'augmenter sa perméabilité puisque le principe actif et le composant lipidique permettant l'ouverture des jonctions serrées arriveraient au même moment au niveau de la membrane. Des premières mesures sur les formules non diluées ont été réalisées. Pour les formules 1 à 5, 10 et 25 %, les mesures obtenues sont du même ordre de grandeur que celles mesurées par *Koga et al.* mais aucune relation entre la concentration en Labrasol® et la taille des particules n'est clairement identifiée. On observe même l'apparition d'une population à 400 nm pour la formule à 5 % qui disparait dans la formule placebo et laisse penser à une influence du principe actif sur la taille des particules. Cependant, les mesures réalisées sur formules après dilution annulent l'hypothèse d'une influence du diamètre des particules puisque les différences observées disparaissent. Par ailleurs, on note une augmentation de taille importante des particules avec la dilution.

Il est remarqué qu'en tenant compte des dilutions effectuées sur cellules, deux concentrations identiques en Labrasol® ont été testées (tableau 15) et ne donnent pas les mêmes résultats de perméabilité. Ce point est un argument en faveur de l'importance de la concentration initiale en principe actif dans l'augmentation du passage membranaire. Le ratio principe actif/Labrasol® s'avère donc important dans les résultats observés. Le tableau 15 rappelle les facteurs d'augmentation observés suivant les ratios principe actif/Labrasol® utilisés.

**Tableau 15 : Augmentation de la perméabilité du baclofène en fonction du ratio principe actif/Labrasol® et de la concentration en Labrasol®**

| Ratio PA/Labrasol® | facteur d'augmentation de Papp | concentration Labrasol® |
|---|---|---|
| 1/1,7 | 0 | 0,01 % |
| 1/8,8 | 0 | 0,05 % |
| 1/13,4 | 7 | 0,03 % |
| 1/18,5 | **20** | 0,05 % |
| 1/55,5 | **28** | 0,125 % |

Ainsi, il semble que l'augmentation de la perméabilité apparente du baclofène n'intervient qu'à partir d'un certain seuil de Labrasol® par rapport à la concentration de baclofène.

Une augmentation de perméabilité apparente est également obtenue pour les microémulsions. Cependant, malgré cette observation, celles-ci ne sont pas retenues en raison de la toxicité mise en évidence sur le tapis cellulaire par perte de l'intégrité membranaire.

Finalement, au vu de l'augmentation importante de perméabilité obtenue avec le Labrasol®, la suite de l'étude s'oriente principalement vers cet excipient notamment pour les essais galéniques.

## b. Essais d'adsorption/Granulation

Les essais de passage sous forme solide par granulation réalisés avec la cellulose microcristalline ne permettent pas l'introduction d'une quantité supérieure à 33 % de *Labrasol®.* Par comparaison visuelle avec l'essai d'adsorption, le grain de Labrasol® semble présenter de meilleures propriétés de par un effet moins « humide », et l'obtention de grain permettant en théorie un meilleur écoulement notamment en vue de futurs essais de compression. L'indice de Carr calculé de 8,14 permet de supposer de bonnes propriétés d'écoulement mais de faibles propriétés de compressibilité. Pourtant une absence d'écoulement est observée en entonnoir normalisé.

D'autres essais d'adsorption sont alors réalisés sur Neusilin®, un silicate possédant une capacité d'adsorption trois fois supérieure à celle de la cellulose microcristalline *(Neusilin application data, www.Neusilin.com).* La technique d'adsorption est préférée car elle permet l'introduction de plus grandes quantités de liquide par rapport à la granulation. En effet cette dernière technique nécessite également l'adsorption d'une certaine quantité d'eau qui réduit le volume disponible pour l'huile. Un mélange sec contenant 60 % de Labrasol® est obtenu ce qui est encourageant et peut permettre l'introduction de cette poudre dans une forme orale pharmaceutique.

## LISTE DES ABREVIATIONS

| | |
|---|---|
| Ac | Acide |
| ATP | Adénosine triphosphate |
| BCS | Biopharmaceutical Classification System |
| BP | British Pharmacopoeia |
| BSA | Bovine sérum albumine |
| C10, C8 | Acide gras composé de 10 ou 8 atomes de carbone |
| cP | centiPoise |
| $d_{10}$, $d_{50}$, $d_{90}$ | Déciles 10,50 et 90% |
| DAG | Diacylglycérol |
| DMEM | Dulbecco'sModifiedEagle Médium |
| DMF | Drug master File |
| DSC | Differential scanning calorimetry |
| E/H | Eau dans huile |
| EMEA | European Agency for the Evaluation of Medicinal Products |
| EP | Pharmacopée Européenne |

(suite)

| | |
|---|---|
| FDA | Food and Drug Administration |
| GRAS | Generally recognized as safe |
| H/E | Huile dans eau |
| HBSS | Hank'sBalanced Salt Solution |
| HEPES | acide 4-(2-hydroxyéthyl)-1-pipérazine éthane sulfonique |
| HLB | Balance hydrophile/lipophile |
| IP | Indice de polydispersité |
| IP3 | Inositol triphosphate |
| JS | Jonctions serrées |
| LC/MS/MS | Chromatographie liquide couplé à la spectophotométrie de masse en tandem |
| LY | Lucifer yellow |
| ME | Microémulsion |
| MTT | bromure de 3-(4,5-dimethylthiazol-2-yl)-2,5-diphenyl tetrazolium |
| N/C | Non connu |
| NF | Norme française |
| OMS | Organisation mondiale de la Santé |
| PA | Principe actif |
| $P_{app}$ | Perméabilité apparente |
| P-gp | P-glycoprotéine |
| PIP2 | phosphatidylinositol 4,5-bisphosphate |
| PLC | Phospholipase C |
| rpm | Rotor per minute (= tours par minute) |
| t ou T | Temps |
| T5, T65, T125 | Temps à 5, 65 ou 125 minutes |
| $T_{amb}$ | Température ambiante |
| TEER | Résistance transépithéliale |
| $T_f$ | Température de fusion |
| USP | United States Pharmacopoeia |
| V0 | Volume vrac |
| V10, V500, V1250 | Volume apparent après 10, 500 ou 1250 tassements |
| ZO | Zonulaoccludens |
| $\rho$ | Masse volumique |

**BIBLIOGRAPHIE**

**Abitec** Fiches techniques // Capmul MCM EP ; Captex 355 EP/NF ; Captex 300 EP/NF ; Captex 200P. - 2012.

Anilkumar P [et al.] A rationalized description on study of intestinal barrier, drug permeability and permeation enhancers [Revue] // Journal of Global Trends in Pharmaceutical Sciences vol 2, n° 4. - 2001. - pp. 431-449.

Boonme P [et al.] Characterization of microemulsion structures in the pseudoternary phase diagram of isopropyl palmitate/water/brij 97:1-butanol [Article] // AAPS PharmSciTech, vol 6, n°2, article 45. - 2006.

Brochette Pascal Emulsification-Elaboration et études des émulsions, J 2150 [En ligne] // Techniques de l'ingénieur. - 2012. - 22 Mars 2012.

Buyukozturk F, Benneya J, C et Carrier R, L Impact of emulsion-based drug delivery systems on intestinal permeability and drug release kinetics [Article] // Journal of Controlled Release, vol 142. - 2010. - pp. 22-30.

Cho S-W, Lee J, S et Choi S-H Enhanced oral bioavailability of poorly absorbed drug. I. Screening absoption carrier for the ceftriaxone complex [Revue] // Journal of Pharmaceutical Sciences, vol 96, n°3. - 2004. - pp. 612-620.

**Choi S-H, Lee J-S et Keith D** Compositions and methods to imptove the oral absorption of antimicrobial agents [Brevet] : EP 1 294 361 B1 - WO 2001/097851. - International, 2011.

Constantinides P, P [et al.] Water-in-oil microemulsions containing medium-chain fatty acids/sats: formulation and intestinal absorption enhancement evaluation [Revue] // Pharmaceutical Research, vol 13, n°2. - 1996. - pp. 210-215.

Constantinides P, P et Scalart J-P Formulation and physical characterization of water-in-oil microemulsions containing long- versus medium-chain glycerides, [Revue] // International Journal of Pharmaceutics, vol 158. - 1997. - pp. 57-68.

Constantinides P, P Lipid microemulsions for improving drug dissolution and oral absorption : Physical and biopharmaceutical aspects [Revue] // Pharmaceutical Research, vol 12, n°11. - 1995. - pp. 1561-1572.

Dahan A, Miller J, M et Amidon G, L Prediction of solubility and permeability class membership: provisional BCS Classification of the world's top oral drug [Revue] // The AAPS Journal, vol 11, n°4. - 2009. - pp. 740-746.

Davis S, S Formulation stratégies for absorption windows [Revue] // Drug Discovery today, vol 10, n°4. - 2005.

DeLuca T, Kaszuba M et Mattison K Optimizing silicone emulsion stability using zeta potential [Article] // American Laboratory News. - Juillet 2006.

Fan Y [et al.] Improved intestinal delivery of salmon calcitonin by water-in-oil microemulsions [Revue] // International Journal of Pharmaceutics, vol 416. - 2011. - pp. 323-330.

Fasinu P [et al.] Diverse approaches for the enhancement of oral drug bioavailability [Revue] // Biopharmaceutics and drug disposition, vol 32. - 2011. - pp. 185-209.

FDA Code of federal regulations title 21, part 172 and 184 [En ligne] // U.S Food and Drug Administration. - 1 Avril 2011. - 9 Mai 2012. - http:/www.accessdata.fda.gov.

**Gattefossé** Lipid excipients for oral bioavailability enhancement.

Gundogdu E, Gonzales Alvarez I et Karasulu E Improvement of effect of water-in-oil microemulsion as an oral delivery system for fexofenadine: in vitro and in vivo studies [Article] // International Journal of Nanomedicine, vol 6. - 2011. - pp. 1631-1640.

ICI Americas Inc The HLB system: a time-saving guide to emulsifier selection [Livre]. - Wilmington : Chemmunique, 1976.

Jannin V, Musakhanian J et Marchaud D Approaches for the development of solid and semi-solid lipid-based formulations [Article] // Advanced drug delivery reviews, vol 60. - 2008. - pp. 734-746.

Koga K, Kawashima S et Murakami M In vitro and in situ evidence for the contribution of labrasol and Gelucire 44/14 on transport of cephalexine and cefoperazone by rat intestine [Revue] // European Journal of Pharmaceutics and biopharmaceutics, vol 54. - 2002. - pp. 311-318.

Leisen C, C [et al.] Lipophilicities of baclofen ester prodrugs correlate with affinities to the ATP-dependant efflux pump P-glycoprotein: relevance for their permeation across the blood-brain barrier? [Revue] // Pharmaceutical Research, vol 20, n°5. - 2003. - pp. 772-778.

Lin Y [et al.]Effects of Labrasol and other pharmaceutical excipients on the intestinal transport and absorption of rhodamin123, a P-Glycoprotein substrate, in rats [Revue] // Biological and Pharmaceutical Bulletin, vol 30, n°7 . - 2007. - pp. 1301-1307.

Maher S [et al.] Safety and efficacy of sodium caprate in promoting oral drug absorption: from in vitro to the clinic [Revue] // Advanced Drug Delivery Reviews, vol 31. - 2009. - pp. 1427-1449.

Merino M [et al.] Evidence of a specialized transport mechanism for the intestinal absorption of baclofen [Revue] // Biopharmaceutics and Dreug disposition, vol 10. - 1989. - pp. 279-297.

Motlekar N [et al.] Oral delivery of low-molecular-weight heparin using sodium caprate as absorption enhancer reaches therapeutic levels [Revue] // Journal of drug targeting, vol 13, n°10. - 2005. - pp. 573-583.

NatafS Le tissu épithélial [En ligne] // Histologie. -11 Mai 2012. - http://histoblog.viabloga.com.

Nekkanti V [et al.] Solid self-microemulsifying formulation for candesartan cilexetil [Revue] // AAPS PharmSciTech, vol 11, n°1. - 2010. - pp. 9-17.

Nollevaux G [et al.]Development of a serum-free co-culture of human intestinal epithelium cell-lines (Caco-2/HT29-5M21) [Article] // BMC cell Biology,vol 7, n°20. - 2006. - pp. 1471-2121.

Oroxcell Protocole d'essais sur cellules Caco-2. - Romainville : [s.n.], 15 Décembre 2011.

Pontier Catherine Coculture de cellules Caco-2/TC7 HT29-MTX: comparaison avec les modèles Caco-2/TC7 et HT 29-MTX [Rapport]. - [s.l.] : Université de Paris XI, centre d'études pharmaceutiques, 1997-1998.

Prajapati H,N, Dalrymple D, M et Serajuddin A,T,M A comparative evaluation of Mono- Di- and Triglyceride of médium chaon fatty acids by lipid/surfactant/water phase diagram, solubility détermination and dispersion testing for application in oharmaceutical dosage form development [Article] // Pharmaceutical research, vol 29. - 2012. - pp. 285-305.

pubchem Sodium Caprate [En ligne] // pubchem. - 10 Avril 2012. - http://pubchem.ncbi.nlm.nih.gov.

Redzic Z Molecular biology of the blood-brain and the blood-cerebrospinal fluid barriers: similarities and differences [Article] // Fluids and barriers of the CNS, vol 8, n°3. - 2011.

Sachs-Barrable S [et al.]Lipid excipients peceol and gelucire 44/14 decrease P-glycoprotein mediated efflux of rhodamine 123 partially due to modifying P-glycoprotein protein expression within Caco-2 cells [Revue] // Journal of Pharmacy and Pharmaceutical Sciences, vol 10, n°3. - 2007. - pp. 319-331.

Sasol Germany GmbH Product information 09.04 // Miglyol 812, 812, 818, 829, 840 neutral oils for pharmaceutical and cosmetics. - 2012.

Sha X [et al.] Effect of self-microemulsifyinf drug delivey systems containing labrasol on tight junctions in Caco-2 cells [Revue] // European Journal of Pharmaceutical Sciences, vol 24. - 2005. - pp. 477-486.

Sigentec Tests de cytotoxicité [En ligne] // site Web Sigentec. - 7 Mai 2012. - http://www.sigentec.com/html/fr/tests/cytotoxicite.html.

Benet L,Z, Broccatelli F et Oprea T,I BDDCS applied to ver 900 drugs [Article] // The AAPS Journal. - 2011.

Kochak G,M [et al.]The pharmacokinetics of baclofen deribed from intestinal infusion [Article] // Clin Pharmacol Ther. -1985. - Vol. 38: pp 251-257.

KMerino M [et al.] Evidence of a specialized transport mechanism for the intestinal absorption of baclofen [Revue] // Biopharmaceutics and Dreug disposition, vol 10. - 1989. - pp. 279-297.

Neusilin Application data [En ligne] // Neusilin.com. - http://www.neusilin.com/library/application_data. - 05 06 2012.

Pharmacopée Européenne 7ème édition // Chapitres 2.09.34-Masse volumique vrac et masse volumique après tassement et 2.09.36-Aptitude à l'écoulement des poudres. - 2012. - Vol. 7.4.

**Revendications**

1. Formulation pharmaceutique orale à base d'une microémulsion entre une solution aqueuse comprenant au moins un principe actif BCS (Biopharmaceutics Classification System) de classe III, ledit principe actif étant le baclofène, et une phase huileuse comprenant un excipient huileux auto-émulsifiable au contact de l'eau, ledit excipient huileux auto-émulsifiable au contact de l'eau étant un mélange de mono-, bi- et triglycérides et d'acides gras estérifiés PEG-8 à raison de 50 à 80 % d'acide caprylique et 20 à 50 % d'acide caprique.

2. Formulation selon la revendication 1, ledit excipient huileux auto-émulsifiable au contact de l'eau présentant un indice HLB supérieur à 12.

3. Formulation selon l'une quelconque des revendications précédentes dans laquelle ladite phase huileuse représente de 1 à 50 % de la micro-émulsion, de préférence de 2 à 30 %, de manière particulièrement préférée de 5 à 25 %.

4. Formulation selon l'une quelconque des revendications précédentes, dans laquelle ladite micro-émulsion est une micro-émulsion huile-dans-eau constituée de ladite phase aqueuse et dudit excipient huileux auto-émulsifiable au contact de l'eau.

5. Formulation selon l'une quelconque des revendications précédentes, ledit excipient huileux auto-émulsifiable au contact de l'eau étant présent dans un rapport de 1/10 à 1/100 en poids par rapport au principe actif.

6. Formulation selon l'une quelconque des revendications précédentes, sous forme liquide comprenant ladite micro-émulsion.

7. Formulation selon l'une quelconque des revendications 1 à 5, sous forme solide.

8. Formulation selon la revendication 7, sous forme de gélule souple ou rigide comprenant ladite microémulsion à l'intérieur de la gélule.

9. Formulation selon la revendication 7, sous forme de comprimé comprenant un support neutre recouvert ou imprégné de ladite microémulsion, séché puis comprimé avec des excipients de compression.

10. Formulation selon l'une quelconque des revendications précédentes pour son utilisation dans le traitement de la dépendance à l'alcool ou le maintien du sevrage des alcooliques.

**Patentansprüche**

1. Orale pharmazeutische Formulierung auf der Grundlage einer Mikroemulsion mit einer wässrigen Lösung, die zumindest einen BCS Wirkstoff (Biopharmaceutics Classification System = Biopharmazeutisches Klassifizierungssystem) der Klasse III umfasst, wobei der Wirkstoff Baclofen ist, und einer Ölphase, umfassend einen öligen Hilfsstoff, der bei Berührung mit Wasser selbstemulgierbar ist, wobei der bei Berührung mit Wasser selbstemulgierbare ölige Hilfsstoff eine Mischung aus Mono-, Di- und Triglyceriden und veresterten Fettsäuren PEG-8 in einer Menge von 50 bis 80 % Caprylsäure und 20 bis 50 % Caprinsäure ist.

2. Formulierung nach Anspruch 1, wobei der bei Berührung mit Wasser selbstemulgierbare ölige Hilfsstoff einen HLB-Wert von mehr als 12 aufweist.

3. Formulierung nach einem beliebigen der vorstehenden Ansprüche, wobei die ölige Phase 1 bis 50 % der Mikroemulsion, vorzugsweise 2 bis 30 %, besonders bevorzugt 5 bis 25 %, ausmacht.

**4.** Formulierung nach einem beliebigen der vorstehenden Ansprüche, wobei die Mikroemulsion eine Ölin-Wasser-Mikroemulsion ist, die aus der wässrigen Phase und dem bei Berührung mit Wasser selbstemulgierbaren öligen Hilfsstoff besteht.

**5.** Formulierung nach einem beliebigen der vorstehenden Ansprüche, wobei der bei Berührung mit Wasser selbstemulgierbare ölige Hilfsstoff in einem Verhältnis von 1:10 bis 1:100 Gew.-% im Verhältnis zum Wirkstoff vorhanden ist.

**6.** Formulierung nach einem beliebigen der vorstehenden Ansprüche in flüssiger Form, umfassend die Mikroemulsion.

**7.** Formulierung nach einem beliebigen der vorstehenden Ansprüche 1 bis 5 in fester Form.

**8.** Formulierung nach Anspruch 7 in Form einer weichen oder starren Kapsel, die die Mikroemulsion im Kapselinnern umfasst.

**9.** Formulierung nach Anspruch 7 in Tablettenform, die einen neutralen Träger umfasst, der mit der Mikroemulsion überzogen oder imprägniert, getrocknet und dann mit Presshilfsstoffen verpresst ist.

**10.** Formulierung nach einem beliebigen der vorstehenden Ansprüche für ihre Verwendung bei der Behandlung von Alkoholabhängigkeit oder der Aufrechterhaltung des Alkoholentzugs.


**Claims**

**1.** An oral pharmaceutical formulation based on a microemulsion between an aqueous solution comprising at least one BCS (Biopharmaceutics Classification System) class III active ingredient, said active ingredient being baclofen, and an oil phase comprising an oil excipient self-emulsifiable in contact with water, said oil excipient self-emulsifiable in contact with water being a mixture of mono-, bi- and triglycerides and of PEG-8 esterified fatty acids in the proportion of 50 to 80 % caprylic acid and 20 to 50 % capric acid.

**2.** The formulation according to claim 1, said oil excipient self-emulsifiable in contact with water having an HLB value higher than 12.

**3.** The formulation according to any one of the preceding claims, wherein said oil phase represents 1 to 50 % of the microemulsion, preferably 2 to 30 %, more preferably 5 to 25 %.

**4.** The formulation according to any one of the preceding claims, wherein said microemulsion is an oil-in-water microemulsion formed of said aqueous phase and of said oil excipient self-emulsifiable in contact with water.

**5.** The formulation according to any one of the preceding claims, said oil excipient self-emulsifiable in contact with water being contained in a ratio of 1:10 to 1:100 by weight relative to the active ingredient.

**6.** The formulation according to any one of the preceding claims, in liquid form comprising said microemulsion.

**7.** The formulation according to any one of claims 1 to 5, in solid form.

**8.** The formulation according to claim 7, in soft or hard capsule form comprising said microemulsion inside the capsule.

**9.** The formulation according to claim 7, in tablet form comprising a neutral carrier coated or impregnated with said microemulsion, dried and compressed with compression excipients.

**10.** The formulation according to any one of the preceding claims, for use thereof in the treatment of alcohol dependence or for the maintaining of alcohol abstinence.

FIGURE 1

FIGURE 2
*JS = Jonctions serrées*

FIGURE 3

*ZO = ZONULA OCCLUDENS*

**FIGURE 4** : COMPOSITION DES MICROEMULSIONS HUILE DANS EAU REALISEES A PARTIR DE MIGLYOL® ET DE LABRASOL®

**FIGURE 5** : COMPOSITION DES MICROEMULSIONS EAU DANS HUILE REALISEES A PARTIR DE CAPTEX® ET DE CAPMUL®

FIGURE 6 : MESURES INITIALES ET FINALES DE RESISTANCE TRANSEPITHELIALE EN FONCTION DES ESSAIS REALISES SUR CELLULES

EP 2 861 216 B1

FIGURE 7 : PERMEABILITES APPARENTE DE LA LUCIFER YELLOW EN FONCTION DES ESSAIS REALISES SUR CELLULES

FIGURE 8 : PERMEABILITES APPARENTE DU PRINCIPE ACTIF (BACLOFENE OU METOPROLOL) EN FONCTION DES ESSAIS REALISES SUR CELLULES

d(0.1): 140.228   µm        d(0.5): 248.981  µm        d(0.9):   414.010   µm

**Distribution granulométrique des particules**

*FIGURE 9* : Distribution granulometrique en volume du grain compose de 33% de Labrasol®

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2010029771 A
- WO 2005025559 A
- EP 1294361 B1
- WO 2001097851 A

**Littérature non-brevet citée dans la description**

- Pharmacopée Européenne. 2012
- **ANILKUMAR P.** A rationalized description on study of intestinal barrier, drug permeability and permeation enhancers. *Journal of Global Trends in Pharmaceutical Sciences,* 2001, vol. 2 (4), 431-449
- **BOONME P.** Characterization of microemulsion structures in the pseudoternary phase diagram of isopropyl palmitate/water/brij 97:1-butanol. *AAPS PharmSciTech,* 2006, vol. 6 (2
- **BROCHETTE PASCAL.** Emulsification-Elaboration et études des émulsions, J 2150. *Techniques de l'ingénieur. - 2012,* 22 Mars 2012
- **BUYUKOZTURK F ; BENNEYA J, C ; CARRIER R, L.** Impact of emulsion-based drug delivery systems on intestinal permeability and drug release kinetics. *Journal of Controlled Release,* 2010, vol. 142, 22-30
- **CHO S-W ; LEE J, S ; CHOI S-H.** Enhanced oral bioavailability of poorly absorbed drug. I. Screening absoption carrier for the ceftriaxone complex. *Journal of Pharmaceutical Sciences,* 2004, vol. 96 (3), 612-620
- **CONSTANTINIDES P, P.** Water-in-oil microemulsions containing medium-chain fatty acids/sats: formulation and intestinal absorption enhancement evaluation. *Pharmaceutical Research,* 1996, vol. 13 (2), 210-215
- **CONSTANTINIDES P, P ; SCALART J-P.** Formulation and physical characterization of water-in-oil microemulsions containing long- versus medium-chain glycerides. *International Journal of Pharmaceutics,* 1997, vol. 158, 57-68
- **CONSTANTINIDES P, P.** Lipid microemulsions for improving drug dissolution and oral absorption : Physical and biopharmaceutical aspects. *Pharmaceutical Research,* 1995, vol. 12 (11), 1561-1572
- **DAHAN A ; MILLER J, M ; AMIDON G, L.** Prediction of solubility and permeability class membership: provisional BCS Classification of the world's top oral drug. *The AAPS Journal,* 2009, vol. 11 (4), 740-746
- **DAVIS S, S.** Formulation stratégies for absorption windows. *Drug Discovery today,* 2005, vol. 10 (4
- **DELUCA T ; KASZUBA M ; MATTISON K.** Optimizing silicone emulsion stability using zeta potential. *American Laboratory News,* Juillet 2006
- **FAN Y.** Improved intestinal delivery of salmon calcitonin by water-in-oil microemulsions. *International Journal of Pharmaceutics,* 2011, vol. 416, 323-330
- **FASINU P.** Diverse approaches for the enhancement of oral drug bioavailability. *Biopharmaceutics and drug disposition,* 2011, vol. 32, 185-209
- FDA Code of federal regulations title 21, part 172 and 184. U.S Food and Drug Administration, 01 Avril 2011
- **GUNDOGDU E ; GONZALES ALVAREZ I ; KARASULU E.** Improvement of effect of water-in-oil microemulsion as an oral delivery system for fexofenadine: in vitro and in vivo studies. *International Journal of Nanomedicine,* 2011, vol. 6, 1631-1640
- The HLB system: a time-saving guide to emulsifier selection. Chemmunique. ICI Americas Inc, 1976
- **JANNIN V ; MUSAKHANIAN J ; MARCHAUD D.** Approaches for the development of solid and semi-solid lipid-based formulations. *Advanced drug delivery reviews,* 2008, vol. 60, 734-746
- **KOGA K ; KAWASHIMA S ; MURAKAMI M.** In vitro and in situ evidence for the contribution of labrasol and Gelucire 44/14 on transport of cephalexine and cefoperazone by rat intestine. *European Journal of Pharmaceutics and biopharmaceutics,* 2002, vol. 54, 311-318
- **LEISEN C, C.** Lipophilicities of baclofen ester prodrugs correlate with affinities to the ATP-dependant efflux pump P-glycoprotein: relevance for their permeation across the blood-brain barrier?. *Pharmaceutical Research,* 2003, vol. 20 (5), 772-778

- **LIN Y.** Effects of Labrasol and other pharmaceutical excipients on the intestinal transport and absorption of rhodamin123, a P-Glycoprotein substrate, in rats. *Biological and Pharmaceutical Bulletin,* 2007, vol. 30 (7), 1301-1307
- **MAHER S.** Safety and efficacy of sodium caprate in promoting oral drug absorption: from in vitro to the clinic. *Advanced Drug Delivery Reviews,* 2009, vol. 31, 1427-1449
- **MERINO M.** Evidence of a specialized transport mechanism for the intestinal absorption of baclofen. *Biopharmaceutics and Dreug disposition,* 1989, vol. 10, 279-297
- **MOTLEKAR N.** Oral delivery of low-molecular-weight heparin using sodium caprate as absorption enhancer reaches therapeutic levels. *Journal of drug targeting,* 2005, vol. 13 (10), 573-583
- **NATAFS.** Le tissu épithélial. *Histologie,* 11 Mai 2012, http://histoblog.viabloga.com.
- **NEKKANTI V.** Solid self-microemulsifying formulation for candesartan cilexetil. *AAPS PharmSciTech,* 2010, vol. 11 (1), 9-17
- **NOLLEVAUX G.** Development of a serum-free co-culture of human intestinal epithelium cell-lines (Caco-2/HT29-5M21). *BMC cell Biology,* 2006, vol. 7 (20), 1471-2121
- **OROXCELL.** *Protocole d'essais sur cellules Caco-2,* 15 Décembre 2011
- Coculture de cellules Caco-2/TC7 HT29. **PONTIER CATHERINE.** MTX: comparaison avec les modèles Caco-2/TC7 et HT 29-MTX. Université de Paris XI, centre d'études pharmaceutiques, 1997
- **PRAJAPATI H,N ; DALRYMPLE D, M ; SERAJUDDIN A,T,M.** A comparative evaluation of Mono- Di- and Triglyceride of médium chaon fatty acids by lipid/surfactant/water phase diagram, solubility détermination and dispersion testing for application in oharmaceutical dosage form development. *Pharmaceutical research,* 2012, vol. 29, 285-305
- **PUBCHEM.** *Sodium Caprate,* 10 Avril 2012, http://pubchem.ncbi.nlm.nih.gov.
- **REDZIC Z.** Molecular biology of the blood-brain and the blood-cerebrospinal fluid barriers: similarities and differences. *Fluids and barriers of the CNS,* 2011, vol. 8 (3
- **SACHS-BARRABLE S.** Lipid excipients peceol and gelucire 44/14 decrease P-glycoprotein mediated efflux of rhodamine 123 partially due to modifying P-glycoprotein protein expression within Caco-2 cells. *Journal of Pharmacy and Pharmaceutical Sciences,* 2007, vol. 10 (3), 319-331
- **SASOL GERMANY GMBH.** Product information 09.04. *Miglyol 812, 812, 818, 829, 840 neutral oils for pharmaceutical and cosmetics,* 2012
- **SHA X.** Effect of self-microemulsifyinf drug delivey systems containing labrasol on tight junctions in Caco-2 cells. *European Journal of Pharmaceutical Sciences,* 2005, vol. 24, 477-486
- **SIGENTEC.** *Tests de cytotoxicité,* 07 Mai 2012, http://www.sigentec.com/html/fr/tests/cytotoxicite.html.
- **BENET L,Z ; BROCCATELLI F ; OPREA T,I.** BDD-CS applied to ver 900 drugs. *The AAPS Journal,* 2011
- **KOCHAK G,M.** The pharmacokinetics of baclofen deribed from intestinal infusion. *Clin Pharmacol Ther.,* 1985, vol. 38, 251-257
- **KMERINO M.** Evidence of a specialized transport mechanism for the intestinal absorption of baclofen. *Biopharmaceutics and Dreug disposition,* 1989, vol. 10, 279-297
- *Neusilin Application data,* 05 Juin 2012, Neusilin.com. - http://www.neusilin.com/library/application_data.
- Masse volumique vrac et masse volumique après tassement et 2.09.36-Aptitude à l'écoulement des poudres. Pharmacopée Européenne. 2012, vol. 7, 4